# EUROPEAN PATENT SPECIFICATION

(11) **EP 4 491 130 B1**
(45) Date of publication and mention of the grant of the patent: **08.04.2026**
(21) Application number: 23306216.5
(22) Date of filing: 13.07.2023
(51) Int. Cl.: A61B 8/12, A61B 8/00

(54) **ULTRASONIC PROBE HAVING QUICK CONNECT HEAD**
ULTRASCHALLSONDE MIT SCHNELLKUPPLUNGSKOPF
SONDE ULTRASONORE À TÊTE DE CONNEXION RAPIDE

(43) Date of publication of application: 15.01.2025
(73) Proprietor: Carestream Dental LLC, Atlanta, GA 30339 (US)
(72) Inventor: CAPRI, Arnaud, 77435 MARNE LA VALLEE CEDEX 2 (FR); LE GRAND, Loïc, 77435 MARNE LA VALLEE CEDEX 2 (FR); BLANCHET, Nicolas, 77435 MARNE LA VALLEE CEDEX 2 (FR)
(74) Representative: Casalonga

(56) References cited:
- WO-A1-2018/149948
- US-A1- 2004 002 658
- US-A1- 2010 249 598
- US-A1- 2022 061 806
- US-B1- 6 659 955

## Description

### FIELD OF THE INVENTION

The present invention relates to the field of intraoral measurement methods and devices for the health care industry. Particularly, but not exclusively, the invention relates to an ultrasonic intraoral wireless handheld device having a quick connect head and a method and mounting assembly for assembling a probe head of the ultrasonic intraoral wireless handheld device to a probe body thereof.

### BACKGROUND OF THE INVENTION

Ultrasound imaging has been adapted for intraoral use in a number of implementations and has been found to have a particular utility for tasks such as measurement of periodontal pocket depth. Conditions such as gingivitis, for example, can be detected by sensing the acoustic response of tissues.

Because of the non-emission of ionizing radiation, ultrasound imaging is inherently safer than ionizing methods and also allows the repeatability of the examination if needed. Ultrasound imaging can be used as a substitute for, or a complement to, various types of radiography (cone beam computed tomography or CBCT, panoramic x-ray, or intraoral x-ray imaging), magnetic resonance imaging (MRI), or nuclear medicine.

Ultrasound imaging may use high-frequency sound waves, typically between 1 to 100 MHz. High-frequency waves being more attenuated than low-frequency waves for a given distance, high-frequency waves are suitable mainly for imaging superficial structures, e.g. for dermatology, or dental imaging. For example, high-frequency sound waves may preferably be between 10 to 50 MHz for periodontal pocket investigation. Conversely, low-frequency waves are suitable for imaging the deepest structures of the body. Moreover, image resolution is also improved when wave frequency is increased.

An ultrasound imaging apparatus generally comprises one or more transducers that act as ultrasound beam emitters and/or ultrasound beam receivers to receive echoes from the emitted signals. In addition, the ultrasound imaging apparatus may comprise various processing and display components used for generating and presenting images from acquired signals. An ultrasound beam emitter generates an ultrasound signal from an electrical signal and conversely, an ultrasound receiver generates electrical pulses from a mechanical ultrasound signal.

Objects in the path of emitted ultrasound signals return a portion of the ultrasound energy back to the transducer which generates electrical signals indicative of the detected structures. Transducers can be of different types among single element transducers or multi-element transducers (annular array, linear array, 2D array, etc.). Furthermore, when a multi-element transducer is used, the emission of ultrasound signals can be delayed in order to enable adaptive focusing. The electronic adaptive focusing makes it possible to increase the resolution depending on the depth of the imaged body structure. It also increases transducer sensitivity which improves image contrast.

The transducer is generally present in a probe head of a probe and is immersed in an acoustic chamber of the probe head that is filled with an internal acoustic coupling material such as a liquid or gel. Since the internal acoustic coupling material may present some degradation issues with age, the probe user may have to remove the probe head from the probe's body and replace the probe head with a new one. Additionally, a probe head may be replaced to adapt the characteristics of the probe head and its transducer to the needs of the practitioner. US6659955 is directed to a modular transducer system comprising a removable scan head.

Therefore, there is a need for an ultrasonic intraoral wireless handheld device having a mechanism for assembling a probe head to a probe body which meets, at least partially, the following constraints:
- removing and replacing the probe head should be done quickly, without tools or with a minimum of tools (e.g., without screwdriver);
- removing and replacing the probe head should avoid the need for plugging and unplugging connection wires (the transducer located in the probe head is generally connected to an electronic main board of the probe body through a coaxial wire);
- removing and replacing the probe head should be done easily by anyone, without requiring specific knowledge, on a plug-and-play basis;
- the assembling mechanism must be low cost;
- the dimensions of the assembling mechanism must be compatible with the dental handheld device constraints (easy handling, easy manipulating, well balanced (i.e., appropriate weight repartition), etc.); and
- the dimensions of the members of the assembling mechanism must be compatible with the manufacturing constraints.

### SUMMARY OF THE INVENTION

The present invention has been devised to address one or more of the foregoing concerns.

In this context, there is provided an ultrasonic handheld device comprising a probe body and a probe head having improved mounting assemblies.

According to a first aspect of the invention, there is provided a mounting assembly for a probe body of an ultrasonic handheld device, the mounting assembly comprising:
a cam guiding member;
a fool-proofing member;
a rotational mechanical crank comprising a crank pin, the rotational mechanical crank having a parking position according to which the crank pin is aligned with the cam guiding member with regard to a mounting direction of a probe head,
   the cam guiding member identifying a reference position of a transducer of the probe head and the fool-proofing member identifying a fastening position of the probe head.

The assembly according to the invention makes it possible to remove and replace reliably a probe head of an intraoral ultrasonic handheld device, at low-cost, while offering a reference position of a transducer and providing reliable electrical contacts and connection.

In an embodiment, the mounting assembly further comprises a positioning and locking member, the positioning and locking member being angularly rigidly connected to the mechanical crank, the positioning and locking member comprising a locking position corresponding to the parking position.

Still in a particular embodiment, the positioning and locking member is mobile along a rotational axis of the mechanical crank, the mounting assembly further comprising a locking member cooperating with the positioning and locking member to lock the mechanical crank in the parking position when a probe head is removed from the probe body.

Still in a particular embodiment, the mounting assembly further comprises a spring member exerting a force on the positioning and locking member to draw the positioning and locking member towards the locking position.

Still in a particular embodiment, the mounting assembly further comprises a position detector to detect an angular position of the positioning and locking member, the angular position corresponding to the locking position.

Still in a particular embodiment, the cam guiding member and/or the fool-proofing member are stationary with regard to the probe body.

Still in a particular embodiment, the mounting assembly further comprises springy or resilient electrical connector pins configured to establish electrical contacts with electrical contact pads of a probe head when the probe head is mounted on the probe body.

According to a second aspect of the invention, there is provided a mounting assembly for a probe head of an ultrasonic handheld device, the mounting assembly being configured to cooperate with the mounting assembly of the probe body described above and comprising:
a fool-proofing member identifying a fastening position of the probe head; and
a cam having a cam groove configured to cooperate with a crank pin of a probe body, the cam being mobile in rotation with regard to the fool-proofing member and being rigidly linked to a transducer.

The assembly according to the invention makes it possible to remove and replace reliably a head of an intraoral handheld device, at low-cost, while offering a reference position of a transducer and providing reliable electrical contacts and connection.

According to a third aspect of the invention, there is provided an ultrasonic handheld device comprising a probe body with a mounting assembly, as described above, and a probe head with a mounting assembly, as described above, the probe head being detachable from the probe body.

The ultrasonic handheld device according to the invention makes it possible to remove and replace reliably a head of an intraoral handheld device, at low-cost, while offering a reference position of a transducer and providing reliable electrical contacts and connection.

In a particular embodiment, the set further comprises the ultrasonic handheld device described above, the probe head being a first probe head, the set further comprising at least one second probe head, the at least one second probe head comprising a mounting assembly as described above, being different from the first probe head, and being attachable to the probe body.

Still in particular embodiments, the at least one second probe head has multiple transducers and/or different types of transducers than the first probe head.

According to a fourth aspect of the invention, there is provided a method for mounting a probe head on a probe body of an ultrasonic handheld device, the probe body comprising a probe body mounting assembly comprising a cam guiding member, a probe body fool-proofing member and a rotational mechanical crank comprising a crank pin, the rotational mechanical crank having a parking position according to which the crank pin is aligned with the cam guiding member with regard to a mounting direction of a probe head, the probe head comprising a probe head mounting assembly comprising a probe head fool-proofing member identifying a fastening position of the probe head and a cam having a cam groove configured to cooperate with the crank pin, the cam being mobile in rotation with regard to the fool-proofing member and being rigidly linked to a transducer, the method comprising
aligning the cam and the cam guiding member;
engaging the cam and the cam guiding member;
aligning the fool-proofing members; and
engaging the fool-proofing members.

The method according to the invention makes it possible to remove and replace reliably a head of an intraoral handheld device, at low-cost, while offering a reference position of a transducer and providing reliable electrical contacts and connection.

In a particular embodiment, engaging the cam and the cam guiding member comprises engaging the crank pin in the cam groove.

Still in a particular embodiment, engaging the fool-proofing members comprises unlocking the mechanical crank.

According to a fifth aspect of the invention, there is provided a method for removing a probe head from a probe body of an ultrasonic handheld device, the probe body comprising a probe body mounting assembly comprising a cam guiding member, a probe body fool-proofing member, and a rotational mechanical crank comprising a crank pin, the rotational mechanical crank having a parking position according to which the crank pin is aligned with the cam guiding member with regard to a mounting direction of a probe head, the probe head comprising a probe head mounting assembly comprising a probe head fool-proofing member identifying a fastening position of the probe head and a cam having a cam groove configured to cooperate with the crank pin, the cam being mobile in rotation with regard to the fool-proofing member and being rigidly linked to a transducer, the method comprising:
aligning the cam and the cam guiding member; and
disengaging the probe head from the probe body.

The method according to the invention makes it possible to remove and replace reliably a head of an intraoral handheld device, at low-cost, while offering a reference position of a transducer and providing reliable electrical contacts and connection.

In a particular embodiment, aligning the cam and the cam guiding member comprises rotating the mechanical crank and detecting a predetermined angular position of the positioning and locking member.

According to a sixth aspect of the invention, there is provided a method for replacing a first probe head of an ultrasonic handheld device by a second probe head, the ultrasonic handheld device comprising a probe head and a probe body, the probe heads being removed and mounted as described above, the second probe head being identical to the first probe head or having different ultrasound measurement characteristics.

The method according to the invention makes it possible to remove and replace reliably a body head of an intraoral handheld device, at low-cost, while offering a reference position of a transducer and providing reliable electrical contacts.

In a particular embodiment, disengaging the probe head from the probe body comprises locking the mechanical crank in the parking position according to which the crank pin is aligned with the cam guiding member.

### BRIEF DESCRIPTION OF THE DRAWINGS

Embodiments of the invention will now be described, by way of example only, and with reference to the following drawings in which:
**Figure 1** is a perspective view of an example of an ultrasonic intraoral wireless handheld device, according to some embodiments of the invention;
**Figure 2** is a perspective view of an example of an ultrasonic intraoral wireless handheld device head, according to some embodiments of the invention, illustrating its mounting assembly;
**Figure 3** is a perspective view of an example of a portion of an ultrasonic intraoral wireless handheld device body, according to some embodiments of the invention, illustrating its mounting assembly;
**Figure 4** illustrates an example of steps for mounting a probe head on a probe body according to some embodiments of the invention;
**Figure 5** is a perspective view of an example of a portion of an ultrasonic intraoral wireless handheld device body and head, according to some embodiments of the invention, illustrating aligning the mounting assemblies of the probe body and head;
**Figure 6** is a cross-sectional perspective view of an example of a portion of an ultrasonic intraoral wireless handheld device body and head, according to some embodiments of the invention, illustrating aligning the cam and the cam guiding member;
**Figure 7** is a cross-sectional perspective view of an example of a portion of an ultrasonic intraoral wireless handheld device body and head, according to some embodiments of the invention, illustrating aligning the fool-proofing members;
**Figure 8** is a cross-sectional perspective view of an example of a portion of an ultrasonic intraoral wireless handheld device body and head, according to some embodiments of the invention, illustrating the assembly of the probe body and head after the probe head is mounted on the probe body;
**Figure 9** illustrates an example of steps for removing a probe head from a probe body, according to some embodiments of the invention; and
**Figure 10** is a schematic block diagram of a processing device for implementation of one or more embodiments of the invention.

### DETAILED DESCRIPTION OF THE INVENTION

The following is a detailed description of particular embodiments of the invention, reference being made to the drawings in which the same reference numerals identify the same elements of structure in each of the Figures.

In the drawings and text that follow, like components are designated with like reference numerals, and similar descriptions concerning components and an arrangement or interaction of components already described are omitted. Where they are used, the terms "first", "second", and so on, do not necessarily denote any ordinal or priority relation, but may simply be used to more clearly distinguish one element from another, unless specified otherwise.

In the context of the present disclosure, the terms "viewer", "operator", and "user" are considered to be equivalent and refer to the viewing practitioner, technician, or other person who acquires, views, and manipulates an ultrasound image, such as a intraoral image, on a display monitor. An "operator instruction," "user instruction," or "viewer instruction" is obtained from explicit commands entered by the viewer, such as by clicking a button on the ultrasound probe or system hardware or by using a computer mouse or by using a touch screen or a keyboard entry.

In the context of the present disclosure, the phrase "in signal communication" indicates that two or more devices and/or components are capable of communicating with each other via signals that travel over some type of signal path. Signal communication may be wired or wireless. The signals may be communication, power, data, or energy signals. The signal paths may include physical, electrical, magnetic, electromagnetic, optical, wired, and/or wireless connections between the first device and/or component and second device and/or component. The signal paths may also include additional devices and/or components between the first device and/or component and second device and/or component.

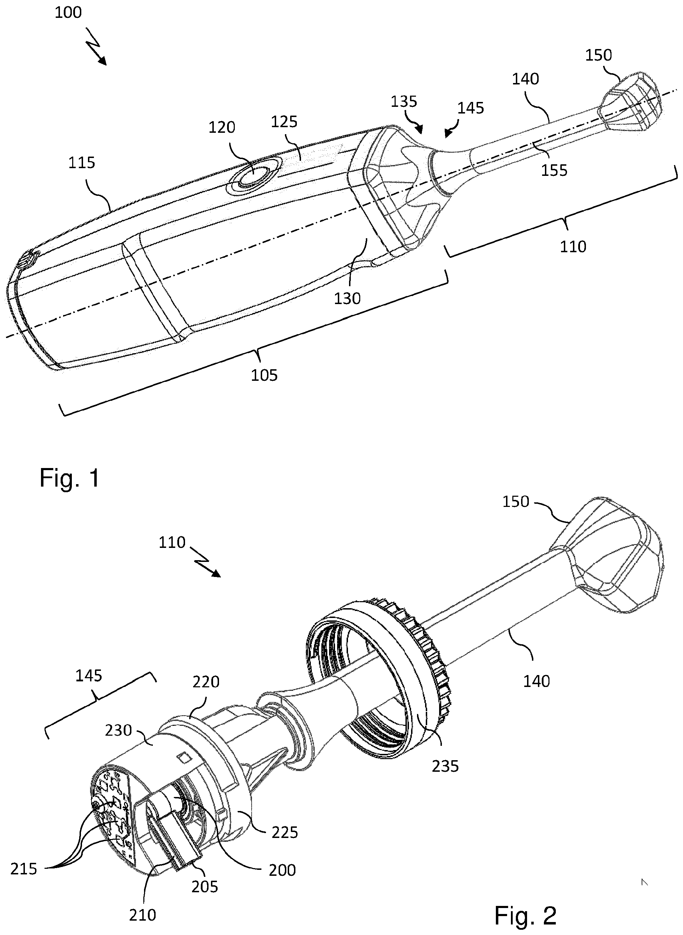

**Figure 1** is a perspective view of an example of an ultrasonic intraoral wireless handheld device 100, also sometimes denoted or referred to herein as a handheld probe or probe, according to some embodiments of the invention. As illustrated, the ultrasonic intraoral wireless handheld device comprises a probe body 105 and a probe head 110 also called a transducer assembly.

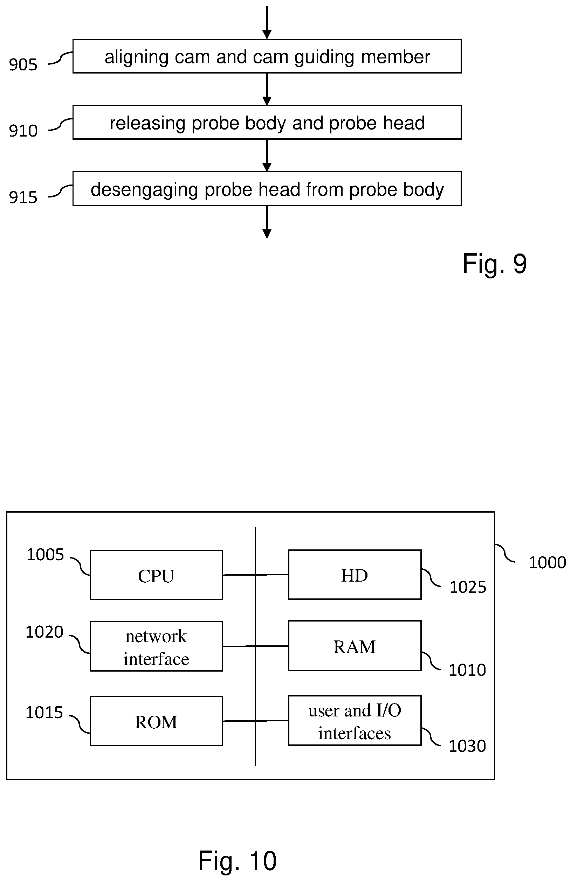

According to this example, probe body 105 comprises a housing 115 that may house a battery and electronic components (not represented), for example a processing device as described by reference to Figure 10. In addition, probe body 105 comprises an interface making it possible for a user to get information from the probe 100 and to provide instructions to the probe 100. For the sake of illustration, probe body 105 may comprise user interface including one or more buttons 120, a display 125, colour LEDs (Light Emitting Diodes) 130 (located under the housing, on one or several faces of the housing, and visible through it), a buzzer, an inertial measurement unit (not represented), and/or a haptic feedback vibrator (not represented). The inertial measurement unit makes it possible to detect user's actions like taps or double-taps, that may be used to interact with the ultrasonic probe. Using button 120 and short, long, and/or repeated clicks and/or using the inertial measurement unit, the user may browse through menus to get information from the probe 100 and/or provide instructions to the probe 100. Probe body 105 also comprises mounting assembly 135 (located under a cover, which may be held in place by an elastic strap, and thus not visible) that makes it possible to mount a probe head 110 on the probe body 105.

As illustrated, probe head 110 comprises a support arm 140 having mounting assembly 145 at one end (cooperating with mounting assembly 135 and thus, located under the same cover), so that probe head 110 may be mounted on a probe body 105, and a transducer chamber 150 at the other end. Transducer chamber 150 houses one or more transducers that may move (for example, by oscillation) to sonicate an object such as gums and other intraoral soft tissues (and possibly to tooth surfaces) of a patient and measure the echo. The transducer is wirelessly connected to electronic components of the probe body or is connected to these electronic components through wires, for example coaxial wires. According to some embodiments, the transducer is mounted at one end of a shaft (not represented) that extends longitudinally through the support arm. A cam may be mounted on the other end of the shaft so as to control the movement of the transducer using a motor located in the probe body. In addition, the shaft may be hollow to allow the passage of a coaxial wire connecting the transducer to electronic components of the probe body 105.

Reference 155 corresponds to the longitudinal axis of the probe body 105 and of the probe head 110, being noted that the longitudinal axis of the probe body 105 may be the same as the longitudinal axis and of the probe head 110 or may be different. In such a case, they may be parallel or not.

Ultrasonic probe 100 may be used for clinical applications such as periodontology, implantology, restorative works, and buccal dermatology.

In particular, ultrasonic probe 100 makes it possible to receive signals from which images of the gums and other intraoral soft tissues (and possibly to tooth surfaces) of a patient may be obtained, these images and/or signals enabling performing one or several of the following tasks:
- detecting morphological abnormalities such as gingival abscess and cancers, and/or
- taking buccal measurements such as measurements of the periodontal pocket depth, of the periodontal gingival margin, of the periodontal attachment level, of the width of an abscess, of anatomical marker distances, etc. for example as described in WO2020148406.

**Figure 2** is a perspective view of an example of an ultrasonic intraoral wireless handheld device head 110, according to some embodiments of the invention, illustrating its mounting assembly, for example the probe head 110 in Figure 1.

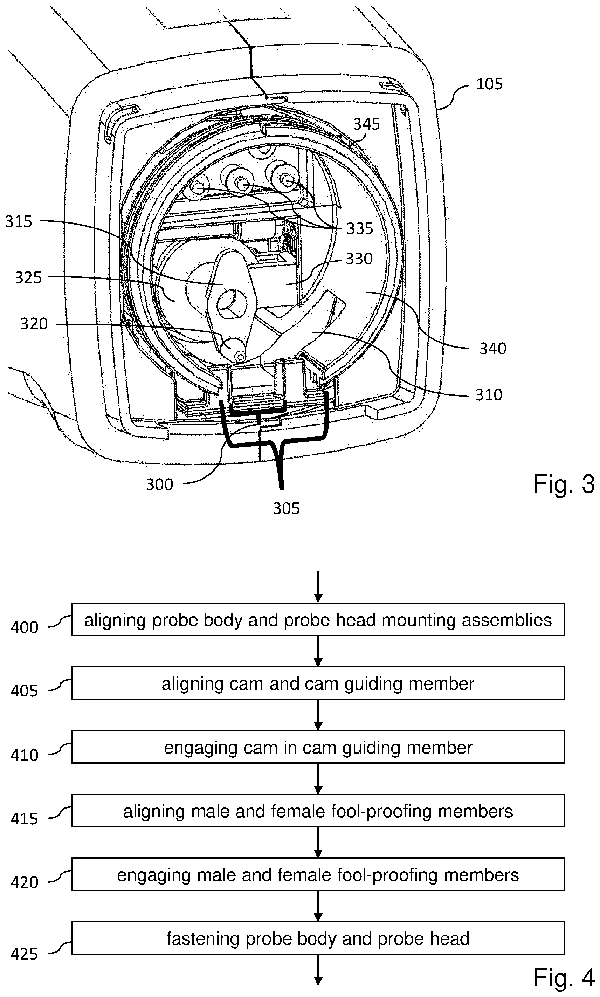

As described above, probe head 110 comprises a support arm 140 having mounting assembly 145 at one end and a transducer chamber 150 at the other end. According to the illustrated example, a rotating shaft 200 extends longitudinally within the support arm 140. The transducer is mounted within the transducer chamber 150 at one end of the rotating shaft 200 and moving cam 205 is mounted at the other end of the rotating shaft 200. Accordingly, the movement of the transducer is controlled by the movement of cam 205 that may be an alternating rotary movement along a given sector. Such a movement may result from a rotational mechanical crank comprising an eccentric or crank pin cooperating with cam groove 210 (for example mechanical crank 315 and crank pin 320 illustrated in Figure 3). According to the illustrated example, cam groove 210 is arranged along cam 205 and is oriented toward the outside of the probe head 110 according to its longitudinal axis.

In addition, probe head 110 comprises electrical contact pads 215 that may be arranged on a printed circuit board. According to some particular embodiments, they are arranged at the end of the probe head 110 (on the mounting assembly side), in a plane perpendicular to the support arm's longitudinal axis. Accordingly, when the probe head 110 is mated with the probe body 105, electrical contact pads 215 may come into contact with electrical connector pins of the probe body 105 (e.g., electrical connector pins 335 in Figure 3), making it possible to establish an electrical connection between the transducer of the probe head 110 and electronic components of the probe body 105.

Still according to some embodiments, probe head 110 comprises flange 220 which may come to rest against the probe body 105 and which may be fastened to the probe body 105 by using threaded ring 235 provided with a shoulder, which lock flange 220 when screwed on the probe body 105. To ensure a particular position of the probe head 110 with regards to the probe body 105, the probe head 110 may be provided with a fool-proofing member, for example a stationary fool-proofing member such as male fool-proofing member 225, that may cooperate with a corresponding female fool-proofing member of the probe body 105, for example a stationary female fool-proofing member (e.g., female fool-proofing member 305 in Figure 3).

According to the illustrated example, mounting assembly 145 comprises a structural member 230 having an external surface having a cylindrical or conical shape, that may cooperate with a corresponding internal shape of a structural member of a probe body 105, to hold the probe head 110 laterally. Still according to the illustrated example, the structural member comprises an opening for cam 205.

**Figure 3** is a perspective view of an example of a portion of an ultrasonic intraoral wireless handheld device body, according to some embodiments of the invention, illustrating its mounting assembly 145, for example a portion of the probe body 105 in Figure 1.

As illustrated, the mounting assembly 145 comprises a cam guiding member 300, for example a stationary cam guiding member, that may be of the U or V shape, to guide moving cam 205 of probe head 110 when probe head 110 is being mounted in probe body 105. According to the illustrated example, the cam guiding member 300 is of the U shape, with a width that is approximately the same as the width of cam 205 (slightly larger so that the cam 205 may be introduced easily in the cam guiding member 300). As illustrated, the cam 205 and/or the cam guiding member 300 may be slightly beveled to facilitate introduction of the cam 205 in the cam guiding member 300. The probe body mounting assembly 145 further comprises a fool-proofing member 305, for example a stationary female fool-proofing member, that may cooperate with male fool-proofing member 225 of probe head 110 so as to position probe head 110 in a predetermined relative position with regard to the probe body 105 when it is mounted in the probe body 105. According to the illustrated example, the fool-proofing member 305 has a rectangular shape, the width of female fool-proofing member 305 being approximately the same as (or slightly larger than) the width of male fool-proofing member 225. To make assembly easier, other shape of the fool-proofing members 225 and 305 may be used, for example a trapezoidal shape. It is also to be noted that the probe body 105 may comprise several fool-proofing members defining several predetermined positions.

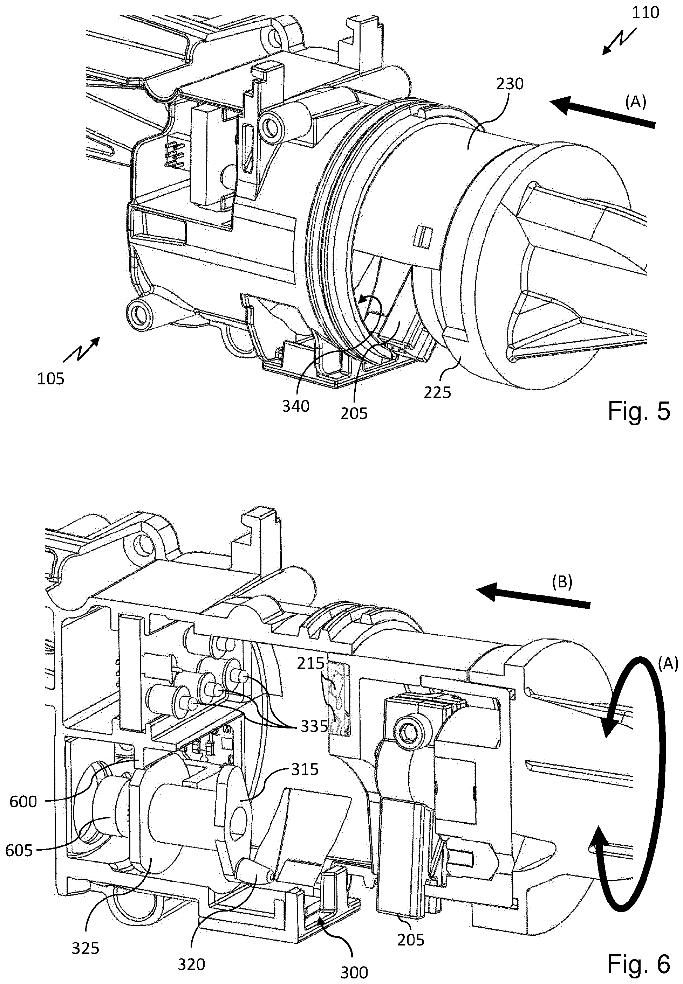

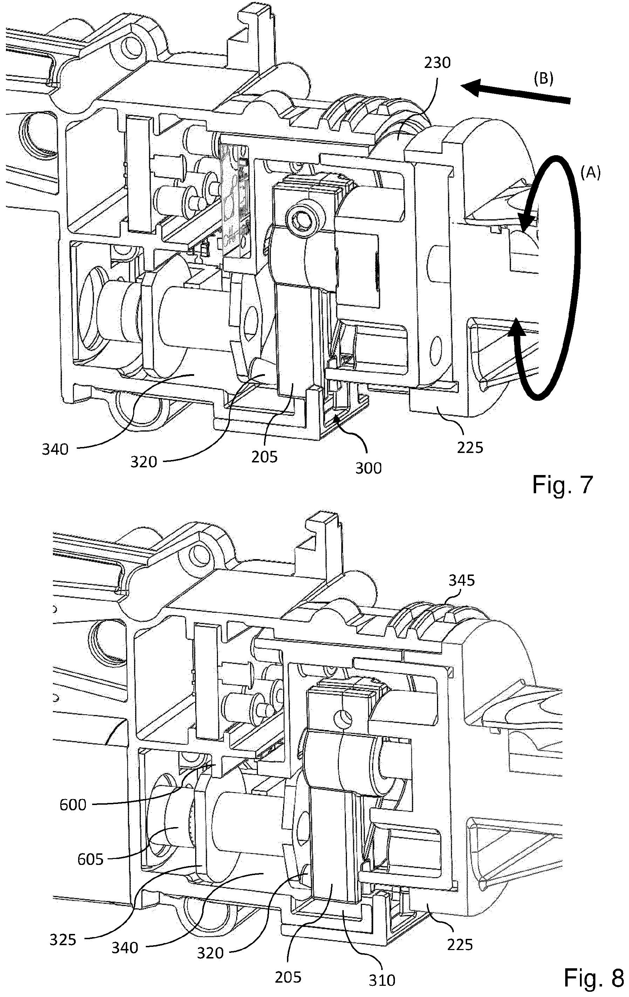

As illustrated, the probe body mounting assembly 145 further comprises a cam clearance window 310 making it possible for cam 205 to move when the probe head 110 is mounted in the probe body 105. To that end, cam clearance window 310 is located behind cam guiding member 300, starting from the external end of the mounting assembly 145 of the probe body 105. To operate cam 205, the mounting assembly 145 further comprises mechanical crank 315 that can rotate around an axis parallel to the rotation axis of cam 205 (when the probe head is mounted in the probe body). Mechanical crank 315 may be driven may a motor (not represented) using a motor shaft. Preferably, mechanical crank 315 may also move slightly along its rotation axis to take either a storage position or an operational position, as described with reference to Figure 8. On its external face (starting from the external end of the mounting assembly 145 of the probe body 105), mechanical crank 315 comprises crank pin 320 that cooperates with cam groove 210 (when the probe head 110 is mounted in the probe body 105) to operate cam 205. Thanks to its shape and, preferably, to a spring (e.g., spring 605 describe in reference to Figure 6), crank pin 320 cooperates efficiently with cam groove 210 to drive it, whatever the dimension variation within dimension tolerances. According to some embodiments, to ensure an efficient cooperation and to facilitate insertion of crank pin 320 into cam groove 210, crank pin 320 is conical. Other shapes may be used, for example a spherical shape.

The mounting assembly 145 further comprises a positioning and locking member 325 that is rigidly coupled or angularly rigidly connected to mechanical crank 315. By cooperating with an associated locking member (or complementary locking member or corresponding locking member), positioning and locking member 325 makes it possible to lock rotation of mechanical crank 315 as described with reference to Figure 8. According to some embodiments, mechanical crank 315 should reach a predetermined position before the probe head 110 is removed and should be locked in this position when the probe head 110 is removed to facilitate next mounting of a probe head 110.

In addition, positioning and locking member 325 makes it possible to identify a predetermined position of the mechanical crank 315, for example using optical detector 330 that is configured to detect a particular element of positioning and locking member 325, such as a hole. Accordingly, an angular position of the positioning and locking member 325, corresponding to the locking position of the mechanical crank 315, may be identified, making it possible to lock the mechanical crank 315 when the probe head 110 is removed.

According to some embodiments, the positioning and locking member 325 comprises a positioning member distinct from the locking member. The positioning member and/or the locking member may be part of the mechanical crank.

As illustrated, the mounting assembly 145 further comprises electrical connector pins 335 that are configured to establish electrical contacts with electrical contact pads of a probe head 110 (when the probe head 110 is mounted in the probe body 105), for example electrical contact pads 215 of probe head 110. According to some particular embodiments, electrical connector pins 335 are springy or resilient (in the direction of the longitudinal axis of the probe body 105) so as to provide reliable electrical contacts when the probe head 110 is mounted in the probe body 105, being observed that springy connector pins 335 make it possible to absorb the mechanical and electrical design deviations (e.g., tilt axis issues between a pin and the corresponding connective pad, distances deviations, etc.).

Still according to the illustrated example, the mounting assembly 145 of the probe body 105 comprises a structural member 340 having an internal surface that is adapted to cooperate with an external surface of a structural member of a probe head 110 and comprises fastening member 345 to fasten the probe body 105 and the probe head 110. According to this example, the internal surface of structural member 340 corresponds to the external surface of the structural member of the mounting assembly 145 of the probe head 110 and fastening member 345 comprises a threaded edge on which threaded ring 235 having a shoulder cooperating with the probe head 110 may be screwed.

It is observed here that there exist other possible shapes for the internal and external surfaces of the structural members of the mounting assemblies 145, for the cam 205 and the cam guiding member300, and for the fool-proofing members 225, 305.

It is also observed here that the position of the angular sector swept by the transducer is mainly determined by the relative position of the transducer with regard to the cam and by the position of the fool-proofing member. Regarding the angular size of the angular sector swept by the transducer, it is mainly determined by the position of the crank pin on the mechanical crank and by the relative position of the rotation axis of the mechanical crank with regard to the rotation axis of the cam. Therefore, the design of the probe head makes it possible to determine the position and the angular size of the angular sector swept by the transducer. Accordingly, different probe heads may be mounted on the same probe body in order to offer different positions and/or different angular sizes of the angular sector swept by the transducer.

**Figure 4** illustrates an example of steps for mounting a probe head 110 in a probe body 105 according to some embodiments of the invention.

As illustrated, a first step (step 400) is directed to aligning the probe body and the probe head, for example probe body 105 and probe head 110, according to their longitudinal axis, the mounting assemblies 145 of the probe body 105 and the probe head 110 facing each other. More precisely, step 400 aims at aligning the mounting assembly components of the probe body 105 and the mounting assembly components of the probe head 110.

Next, the cam, e.g., cam 205 of probe head 110, and the cam guiding member, e.g., cam guiding member 300 of probe body 105, are aligned (step 405). This can be done, for example, by rotating one of the probe body 105 and the probe head 110 with regard to the other. Once the cam 205 and the cam guiding member 300 are aligned, the cam 205 is engaged in and mated with the cam guiding member 300 (step 410), for example by moving the probe body 105 and the probe head 110 towards one another along their longitudinal axis.

Next, once the cam 205 is engaged in the cam guiding member 300, the male and female fool-proofing members, e.g., male and female fool-proofing members 225 and 305 of probe head 110 and probe body 105, are aligned (step 415). Again, this can be done, for example, by rotating one of the probe body 105 and the probe head 110 with regard to the other, it being observed that the cam 205 being engaged in the cam guiding member 300, its relative position with regard to the probe body 105 does not change when the probe body 105 or the probe head 110 is rotated in order to align the fool-proofing members 225, 305. Once the fool-proofing members 225, 305 are aligned, the male fool-proofing member 225 is engaged in the female fool-proofing member 305 (step 420), for example by moving the probe body 105 and the probe head 110 towards one another along their longitudinal axes.

When engaging the male fool-proofing member 225 in the female fool-proofing member 305, the crank pin, e.g., crank pin 320 of probe body 105, engages in the cam groove, e.g., cam groove 210 of probe head 110, it being noted that the mechanical crank, e.g., mechanical crank 315 of probe body 105, is locked (as described here after), in the absence of probe head 110, in a position according to which the crank pin 320 is aligned with the cam groove 210 when the cam 205 is engaged in the cam guiding member 300.

Next, the probe head 110 may be fastened to the probe body 105 (step 425), for example by screwing, on the probe body 105, a threaded ring (e.g., threaded ring 235) having a shoulder cooperating with the probe head 110.

Steps 400, 405, 415, and 420 are illustrated with Figures 5 to 8.

**Figure 5** is a perspective view of an example of a portion of an ultrasonic intraoral wireless handheld device body and head, according to some embodiments of the invention. Figure 5 illustrates step 400 in Figure 4 of aligning the mounting assembly components of the probe body 105 and head 110.

As illustrated, the probe body 105 and the probe head 110 are aligned along their longitudinal axes so that their mounting assembly components face each other, making it possible to insert the structural member 230 of the probe head 110 within the structural member 340 of the probe body 105, as illustrated with the arrow referenced A.

**Figure 6** is a cross-sectional perspective view of an example of a portion of an ultrasonic intraoral wireless handheld device body and head, according to some embodiments of the invention. Figure 6 illustrates step 405 in Figure 4 of aligning the cam 205 and the cam guiding member 300. As described above, aligning the cam 205 and the cam guiding member 300 may be done, for example, by rotating one of the probe body 105 and the probe head 110 with regard to the other until the cam 205 faces the cam guide member 300, as illustrated with arrow A.

Once the cam 205 and the cam guiding member 300 are aligned, the cam 205 is engaged in the cam guiding member 300, for example by moving the probe body 105 and the probe head 110 towards one another along their longitudinal axes, as illustrated with arrow *B*.

According to some embodiments, the mechanical crank 315 is in a parking position (or rest position), when the probe head 110 is mounted in the probe body 105. In such a parking position, the crank pin 320 is aligned with the cam guiding member 300 and thus, engaging the cam 205 in the cam guiding member 300 leads to engaging the crank pin 320 in the cam groove 210.

It is observed that when the cam 205 is engaged in the cam guiding member 300, the electrical connector pins 335 and the electrical contact pads 215 may be misaligned.

**Figure 7** is a cross-sectional perspective view of an example of a portion of an ultrasonic intraoral wireless handheld device body 105 and head 110, according to some embodiments of the invention. Figure 7 illustrates step 415 in Figure 4 of aligning the fool-proofing members 225, 305. As described above, aligning the fool-proofing members 225, 305 may be done, for example, by rotating one of the probe body 105 and the probe head 110 with regard to the other until the male fool-proofing member 225 faces the female fool-proofing member 305, as illustrated with arrow *A*.

It is noted here that according to the illustrated example, when aligning the fool-proofing members 225, 305, the cam 205 is engaged in the cam guiding member 300 and thus, the transducer is stationary with respect to the probe body 105.

Once the fool-proofing members 225, 305 are aligned, the male fool-proofing member 225 is engaged in the female fool-proofing member 305, for example by moving the probe body 105 and the probe head 110 towards one another along their longitudinal axes, as illustrated by arrow *B*.

It is observed that when the male fool-proofing member 225 is engaged in the female fool-proofing member 305, the electrical connector pins 335 and the electrical contact pads 215 are aligned. The electrical contact between the electrical connector pins 335 and the electrical contact pads 215 is established by the force exerted by the probe head 110 toward the probe body 105 and the elasticity of the electrical connector pins 335.

In addition, when the male fool-proofing member 225 is engaged in the female fool-proofing member 305, the crank pin 320 butts up against the cam groove. According to some embodiments, the force exerted by the cam 205 on the crank pin 320 (and thus on the mechanical crank 315 and on the positioning and locking member 325) moves the mechanical crank 315 along its rotational axis, from a locking position illustrated in Figure 7 to an unlocking position illustrated in Figure 8.

Still according to some embodiments, the mechanical crank 315 is spring mounted on its rotational axis, with force exerted towards the end of the probe mounting assembly 145 of the probe body 105, which makes it possible to:
- provide a mechanical gap between the crank 315 and the cam groove 210 to compensate for minor misadjustments;
- keep contact, by pressure, between the crank pin 320 and the cam groove 210; and
- provide a locking position of the mechanical crank 315 once the probe head 110 is removed. It allows maintaining the physical position of the crank pin 320 when the probe head 110 is removed and ensure the correct alignment between the crank pin 320 and the cam groove 210 when mounting a probe head 110 in the probe body 105. The positioning may be ensured by a rotational lock mechanism (or mechanical stop) once the probe head 110 is removed from the probe body 105.

**Figure 8** is a cross-sectional perspective view of an example of a portion of an ultrasonic intraoral wireless handheld device body and head, according to some embodiments of the invention. Figure 8 illustrates the assembly of the probe body 105 and head 110 after the probe head 110 is mounted in the probe body 105 (i.e., between steps 420 and 425 in Figure 4). For the sake of clarity, the printed circuit board comprising the electrical contact pads 215 (in the probe head 110) is not represented.

As illustrated, the force exerted by the cam 205 on the positioning and locking member 325 through the crank pin 320 and the mechanical crank 315 has shifted the positioning and locking member 325 from the locking member 600 (that is here rigidly mounted in the probe body 105), also denoted associated, complementary, or corresponding locking member, enabling the positioning and locking member 325 (and the mechanical crank 315) to rotate along the mechanical crank axis. Spring 605 exerts a biasing force towards the end of the mounting assembly components of the probe body 105 so that when the probe head 110 is removed, the positioning and locking member 325 is aligned with locking member 600, preventing the positioning and locking member 325 (and the mechanical crank 315) to rotate along the mechanical crank axis.

As illustrated, cam 205 faces cam clearance window 310 when the cam 205 has shifted positioning and locking member 325 from the locking member 600, enabling movement of the transducer.

It is observed that the position of the cam guiding member 300 makes it possible to mount the probe head 110 in such a way that the relative position of the transducer with regard to the probe body 105 is predetermined and thus, may be controlled precisely.

**Figure 9** illustrates an example of steps for removing a probe head from a probe body, according to some embodiments of the invention.

As illustrated, a first step is directed to positioning the cam 205 in a predetermined position (step 900), for example a position according to which the cam 205 is aligned with the cam guiding member 300. According to some embodiments, aligning the cam 205 and the cam guiding member 300 comprises rotating the cam 205 until detecting the predetermined position, that may be detected using a sensor such as optical detector 330 and a particular element of positioning and locking member 325 such as a hole (not visible on the drawings). Rotating the cam 205 may be done with an electrical motor that is controlled by a processing unit 1005 such as the one illustrated in Figure 10. Likewise, detecting the predetermined position may be carried out by analyzing, in the processing unit 1005, a signal obtained from the sensor.

Next, the probe head 110 is released from the probe body 105 (step 905), for example by unscrewing a threaded ring (e.g., threaded ring 235) provided with a shoulder, which lock flange 220 when screwed on the probe body 105.

Next, the probe head 110 may be removed from the probe body 105 (step 910).

It is observed that when the probe head 110 is removed from the probe body 105, the force exerted on the crank pin 320 stops and thus, positioning and locking member 325 reaches a locking position due to the force exerted by spring 605 (i.e., positioning and locking member 325 and locking member 600 are aligned, preventing positioning and locking member 325 from rotating).

Next, another probe head 110 (or the same probe head 110) may be mounted in the probe body 105 as described by reference to Figure 4.

**Figure 10** is a schematic block diagram of a processing device for implementation of one or more embodiments of the invention, in particular for controlling rotation of a mechanical crank 315 and/or detecting a predetermined position of the mechanical crank 315.

Computing device 1000 comprises a communication bus connected to:
- a central processing unit 1005, such as a microprocessor, denoted CPU 1005;
- a random access memory 1010, denoted RAM 1010, for storing the executable code of the method of embodiments of the invention as well as the registers adapted to record variables and parameters necessary for implementing a method for training an automatic tooth charting system according to embodiments of the invention, the memory capacity of which can be expanded by an optional RAM connected to an expansion port for example;
- a read-only memory 1015, denoted ROM 1015, for storing computer programs for implementing embodiments of the invention;
- a user interface and/or an input/output interface 1030 which can be used for receiving inputs from a user, displaying information to a user, and/or receiving/sending data from/to external devices; and
- a network interface 1020 typically connected to a communication network over which digital data can be transmitted or received for receiving/sending data from/to remote devices. The network interface 1020 can be a single network interface, or composed of a set of different network interfaces (for instance, wired and/or wireless interfaces, or different kinds of wired or wireless interfaces). Data packets are written to the network interface for transmission or are read from the network interface for reception under the control of the software application running in the CPU 1005.

Optionally, the communication bus of computing device 1000 may be connected to a hard disk 1025 denoted HD 1025 used as a mass storage device.

The executable code may be stored either in read-only memory 1015, on hard disk 1025 or on a removable digital medium such as for example a disk. According to a variant, the executable code of the programs can be received by means of a communication network, via the network interface 1020, in order to be stored in one of the storage means of the computing device 200, such as hard disk 1025, before being executed.

Central processing unit 1005 is adapted to control and direct the execution of the instructions or portions of software code of the program or programs according to embodiments of the invention, the instructions being stored in one of the aforementioned storage means. After powering on, CPU 1005 is capable of executing instructions from main RAM memory 1010 relating to a software application after those instructions have been loaded from ROM 1015 or from hard-disk 1025 for example. Such a software application, when executed by CPU 1005, causes the steps of the algorithms herein disclosed to be performed.

Any step of the algorithm herein disclosed may be implemented in software by execution of a set of instructions or program by a programmable computing machine, such as a PC ("Personal Computer"), laptop computer, tablet, smartphone, a DSP ("Digital Signal Processor") or a microcontroller; or else implemented in hardware by a machine or a dedicated component, such as an FPGA ("Field-Programmable Gate Array") or an ASIC ("Application-Specific Integrated Circuit").

Although the present disclosure has been described herein above with reference to some specific embodiments, the present invention is not limited to these specific embodiments, and modifications will be apparent to a person skilled in the art which lie within the scope of the present invention.

Many further modifications and variations will suggest themselves to those versed in the art upon making reference to the foregoing illustrative embodiments, which are given by way of example only and which are not intended to limit the scope of the invention, that being determined solely by the appended claims. In particular, the different features from different embodiments may be interchanged, where appropriate.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used.

## Claims

1. A mounting assembly for a probe body of an ultrasonic handheld device, the mounting assembly comprising:
a cam guiding member (300);
a fool-proofing member (305);
a rotational mechanical crank (315) comprising a crank pin (320), the rotational mechanical crank having a parking position according to which the crank pin is aligned with the cam guiding member with regard to a mounting direction of a probe head,
the cam guiding member identifying a reference position of a transducer of the probe head and the fool-proofing member identifying a fastening position of the probe head.

2. The mounting assembly of claim 1, further comprising a positioning and locking member (325), the positioning and locking member being angularly rigidly connected to the mechanical crank, the positioning and locking member comprising a locking position corresponding to the parking position.

3. The mounting assembly of claim 2, wherein the positioning and locking member (325) is mobile along a rotational axis of the mechanical crank, the mounting assembly further comprising a locking member (600) cooperating with the positioning and locking member to lock the mechanical crank in the parking position when a probe head is removed from the probe body.

4. The mounting assembly of claim 2 or claim 3, further comprising a spring member (605) exerting a force on the positioning and locking member to draw the positioning and locking member towards the locking position.

5. The mounting assembly of any one of claims 2 to 4, further comprising a position detector to detect an angular position of the positioning and locking member, the angular position corresponding to the locking position.

6. The mounting assembly of any one of claims 1 to 5, wherein the cam guiding member and/or the fool-proofing member are stationary with regard to the probe body.

7. The mounting assembly of any one of claims 1 to 6, further comprising springy electrical connector pins configured to establish electrical contacts with electrical contact pads of a probe head when the probe head is mounted in the probe body.

8. A mounting assembly for a probe head of an ultrasonic handheld device, the mounting assembly being configured to cooperate with the mounting assembly of the probe body of any one of claims 1 to 6 and comprising:
a fool-proofing member (225) identifying a fastening position of the probe head; and
a cam (200) having a cam groove (210) configured to cooperate with a crank pin of a probe body, the cam being mobile in rotation with regard to the fool-proofing member and being rigidly linked to a transducer.

9. An ultrasonic handheld device comprising a probe body with a mounting assembly according to any one of claims 1 to 7 and a probe head with a mounting assembly according to claim 8, the probe head being detachable from the probe body.

10. A set comprising the ultrasonic handheld device of claim 9, the probe head being a first probe head, the set further comprising at least one second probe head, the at least one second probe head comprising a mounting assembly according to claim 8, being different from the first probe head, and being attachable to the probe body.

11. The set of claim 10, wherein the at least one second probe head has multiple transducers and/or a different type of transducer than the first probe head.

12. A method for mounting a probe head in a probe body of an ultrasonic handheld device, the probe body comprising a probe body mounting assembly comprising a cam guiding member (300), a probe body fool-proofing member (305), and a rotational mechanical crank (315) comprising a crank pin (320), the rotational mechanical crank having a parking position according to which the crank pin is aligned with the cam guiding member with regard to a mounting direction of a probe head, the probe head comprising a probe head mounting assembly comprising a probe head fool-proofing member (225) identifying a fastening position of the probe head and a cam (200) having a cam groove (210) configured to cooperate with the crank pin, the cam being mobile in rotation with regard to the fool-proofing member and being rigidly linked to a transducer, the method comprising
aligning (405) the cam and the cam guiding member;
engaging (410) the cam and the cam guiding member;
aligning (415) the fool-proofing members; and
engaging (420) the fool-proofing members.

13. The method of claim 12, wherein engaging the cam and the cam guiding member comprises engaging the crank pin in the cam groove.

14. The method of claim 12 or claim 13, wherein engaging the fool-proofing members comprises unlocking the mechanical crank.

15. A method for removing a probe head from a probe body of an ultrasonic handheld device, the probe body comprising a probe body mounting assembly comprising a cam guiding member (300), a probe body fool-proofing member (305), and a rotational mechanical crank (315) comprising a crank pin (320), the rotational mechanical crank having a parking position according to which the crank pin is aligned with the cam guiding member with regard to a mounting direction of a probe head, the probe head comprising a probe head mounting assembly comprising a probe head fool-proofing member (225) identifying a fastening position of the probe head and a cam (200) having a cam groove (210) configured to cooperate with the crank pin, the cam being mobile in rotation with regard to the fool-proofing member and being rigidly linked to a transducer, the method comprising:
aligning (905) the cam and the cam guiding member; and
disengaging (915) the probe head from the probe body.

16. The method of claim 15, wherein aligning the cam and the cam guiding member comprises rotating the mechanical crank and detecting a predetermined angular position of the positioning and locking member.

17. The method of claim 15 or claim 16, wherein disengaging the probe head from the probe body comprises locking the mechanical crank in the parking position according to which the crank pin is aligned with the cam guiding member.

18. A method for replacing a first probe head of an ultrasonic handheld device by a second probe head, the ultrasonic handheld device comprising a probe head and a probe body, the method comprising:
removing the first probe head from the probe body according to any one of claims 15 to 17; and
mounting the second probe head on the probe body according to any one of claims 12 to 14,
the second probe head being identical to the first probe head or having different ultrasound measurement characteristics.

## Patentansprüche

1. Befestigungsbaugruppe für einen Sondenkörper einer Ultraschallhandvorrichtung, wobei die Befestigungsbaugruppe Folgendes umfasst:
ein Nockenführungselement (300);
ein Fehlbedienungsschutzelement (305);
eine drehmechanische Kurbel (315), die einen Kurbelstift (320) umfasst, wobei die drehmechanische Kurbel eine Parkposition aufweist, in der der Kurbelstift hinsichtlich einer Befestigungsrichtung eines Sondenkopfs mit dem Nockenführungselement ausgerichtet ist,
wobei das Nockenführungselement eine Referenzposition eines Wandlers des Sondenkopfs identifiziert, und das Fehlbedienungsschutzelement eine Befestigungsposition des Sondenkopfs identifiziert.

2. Befestigungsbaugruppe nach Anspruch 1, ferner umfassend ein Positionierungs- und Verriegelungselement (325), wobei das Positionierungs- und Verriegelungselement winkelfest mit der mechanischen Kurbel verbunden ist, wobei das Positionierungs- und Verriegelungselement eine der Parkposition entsprechende Verriegelungsposition umfasst.

3. Befestigungsbaugruppe nach Anspruch 2, wobei das Positionierungs- und Verriegelungselement (325) entlang einer Drehachse der mechanischen Kurbel beweglich ist, wobei die Befestigungsbaugruppe ferner ein Verriegelungselement (600) umfasst, das mit dem Positionierungs- und Verriegelungselement zusammenarbeitet, um die mechanische Kurbel in der Parkposition zu verriegeln, wenn ein Sondenkopf von dem Sondenkörper entfernt wird.

4. Befestigungsbaugruppe nach Anspruch 2 oder 3, ferner umfassend ein Federelement (605), das eine Kraft auf das Positionierungs- und Verriegelungselement ausübt, um das Positionierungs- und Verriegelungselement in Richtung der Verriegelungsposition zu ziehen.

5. Befestigungsbaugruppe nach einem der Ansprüche 2 bis 4, ferner umfassend einen Positionsdetektor zum Erfassen einer Winkelposition des Positionier- und Verriegelungselements, wobei die Winkelposition der Verriegelungsposition entspricht.

6. Befestigungsbaugruppe nach einem der Ansprüche 1 bis 5, wobei das Nockenführungselement und/oder das Fehlbedienungsschutzelement in Bezug auf den Sondenkörper stationär sind.

7. Befestigungsbaugruppe nach einem der Ansprüche 1 bis 6, ferner umfassend federnde elektrische Anschlussstifte, die so eingerichtet sind, dass sie elektrische Kontakte mit elektrischen Kontaktstellen eines Sondenkopfs herstellen, wenn der Sondenkopf in dem Sondenkörper befestigt ist.

8. Befestigungsbaugruppe für einen Sondenkopf einer Ultraschallhandvorrichtung, wobei die Befestigungsbaugruppe so eingerichtet ist, dass sie mit der Befestigungsbaugruppe des Sondenkörpers nach einem der Ansprüche 1 bis 6 zusammenarbeitet, und umfassend:
ein Fehlbedienungsschutzelement (225), das eine Befestigungsposition des Sondenkopfs identifiziert; und
einen Nocken (200), der eine Nockennut (210) aufweist, die so eingerichtet ist, dass sie mit einem Kurbelstift eines Sondenkörpers zusammenarbeitet, wobei der Nocken in Bezug auf das Fehlbedienungsschutzelement drehbeweglich ist und
starr mit einem Wandler verbunden ist.

9. Ultraschallhandvorrichtung, umfassend einen Sondenkörper mit einer Befestigungsbaugruppe nach einem der Ansprüche 1 bis 7 und einen Sondenkopf mit einer Befestigungsbaugruppe nach Anspruch 8, wobei der Sondenkopf von dem Sondenkörper abnehmbar ist.

10. Satz, der die Ultraschallhandvorrichtung nach Anspruch 9 umfasst, wobei der Sondenkopf ein erster Sondenkopf ist, wobei der Satz ferner mindestens einen zweiten Sondenkopf umfasst, wobei der mindestens eine zweite Sondenkopf eine Befestigungsbaugruppe nach Anspruch 8 umfasst, die sich von dem ersten Sondenkopf unterscheidet und an dem Sondenkörper befestigt werden kann.

11. Satz nach Anspruch 10, wobei der mindestens eine zweite Sondenkopf mehrere Wandler und/oder einen anderen Typ von Wandler als der erste Sondenkopf aufweist.

12. Verfahren zum Befestigen eines Sondenkopfs in einem Sondenkörper einer Ultraschallhandvorrichtung, wobei der Sondenkörper eine Sondenkörper-Befestigungsbaugruppe umfasst, die ein Nockenführungselement (300), ein Sondenkörper-Fehlbedienungsschutzelement (305) und eine drehmechanische Kurbel (315) umfasst, die einen Kurbelstift (320) umfasst, wobei die drehmechanische Kurbel eine Parkposition aufweist, in der der Kurbelstift mit dem Nockenführungselement in Bezug auf eine Befestigungsrichtung eines Sondenkopfs ausgerichtet ist, wobei der Sondenkopf eine Sondenkopf-Befestigungsbaugruppe umfasst, die ein Sondenkopf-Fehlbedienungsschutzelement (225), das eine Befestigungsposition des Sondenkopfs identifiziert, und einen Nocken (200) umfasst, der eine Nockennut (210) aufweist, die so eingerichtet ist, dass sie mit dem Kurbelstift zusammenarbeitet, wobei der Nocken in Bezug auf das Fehlbedienungsschutzelement drehbeweglich ist und starr mit einem Wandler verbunden ist, wobei das Verfahren Folgendes umfasst:
Ausrichten (405) des Nockens und des Nockenführungselements;
Einrasten (410) des Nockens und des Nockenführungselements;
Ausrichten (415) der Fehlbedienungsschutzelemente; und
Einrasten (420) der Fehlbedienungsschutzelemente.

13. Verfahren nach Anspruch 12, wobei das Einrasten des Nockens und des Nockenführungselements das Einrasten des Kurbelstifts in die Nockennut umfasst.

14. Verfahren nach Anspruch 12 oder Anspruch 13, wobei das Einrasten der Fehlbedienungsschutzelemente das Entriegeln der mechanischen Kurbel umfasst.

15. Verfahren zum Entfernen eines Sondenkopfs von einem Sondenkörper einer Ultraschallhandvorrichtung, wobei der Sondenkörper eine Sondenkörper-Befestigungsbaugruppe umfasst, die ein Nockenführungselement (300), ein Sondenkörper-Fehlbedienungsschutzelement (305) und eine drehmechanische Kurbel (315) umfasst, die einen Kurbelstift (320) umfasst, wobei die drehmechanische Kurbel eine Parkposition aufweist, in der der Kurbelstift mit dem Nockenführungselement in Bezug auf eine Befestigungsrichtung eines Sondenkopfs ausgerichtet ist, wobei der Sondenkopf eine Sondenkopf-Befestigungsbaugruppe umfasst, die ein Sondenkopf-Fehlbedienungsschutzelement (225), das eine Befestigungsposition des Sondenkopfs identifiziert, und einen Nocken (200) umfasst, der eine Nockennut (210) aufweist, die so eingerichtet ist, dass sie mit dem Kurbelstift zusammenarbeitet, wobei der Nocken in Bezug auf das Fehlbedienungsschutzelement drehbeweglich ist und starr mit einem Wandler verbunden ist, wobei das Verfahren Folgendes umfasst:
Ausrichten (905) des Nockens und des Nockenführungselements; und
Ausrasten (915) des Sondenkopfs von dem Sondenkörper.

16. Verfahren nach Anspruch 15, wobei das Ausrichten des Nockens und des Nockenführungselements das Drehen der mechanischen Kurbel und das Erfassen einer vorbestimmten Winkelposition des Positionierungs- und Verriegelungselements umfasst.

17. Verfahren nach Anspruch 15 oder Anspruch 16, wobei das Ausrasten des Sondenkopfs von dem Sondenkörper das Verriegeln der mechanischen Kurbel in der Parkposition umfasst, in der der Kurbelstift mit dem Nockenführungselement ausgerichtet ist.

18. Verfahren zum Ersetzen eines ersten Sondenkopfs einer Ultraschallhandvorrichtung durch einen zweiten Sondenkopf, wobei die Ultraschallhandvorrichtung einen Sondenkopf und einen Sondenkörper umfasst, wobei das Verfahren Folgendes umfasst:
Entfernen des ersten Sondenkopfs aus dem Sondenkörper nach einem der Ansprüche 15 bis 17; und
Befestigen des zweiten Sondenkopfs an dem Sondenkörper nach einem der Ansprüche 12 bis 14,
wobei der zweite Sondenkopf identisch mit dem ersten Sondenkopf ist oder andere Ultraschallmesseigenschaften aufweist.

## Revendications

1. Ensemble de montage pour un corps de sonde d'un dispositif portatif à ultrasons, l'ensemble de montage comprenant :
un organe de guidage de came (300) ;
un organe de détrompage (305) ;
une manivelle mécanique rotative (315) comprenant un maneton (320), la manivelle mécanique rotative présentant une position de repos selon laquelle le maneton est aligné sur l'organe de guidage de came par rapport à une direction de montage d'une tête de sonde,
l'organe de guidage de came identifiant une position de référence d'un transducteur de la tête de sonde et l'organe de détrompage identifiant une position de fixation de la tête de sonde.

2. Ensemble de montage selon la revendication 1, comprenant en outre un organe de positionnement et de verrouillage (325), l'organe de positionnement et de verrouillage étant solidarisé angulairement à la manivelle mécanique, l'organe de positionnement et de verrouillage comprenant une position de verrouillage correspondant à la position de repos.

3. Ensemble de montage selon la revendication 2, dans lequel l'organe de positionnement et de verrouillage (325) est mobile le long d'un axe de rotation de la manivelle mécanique, l'ensemble de montage comprenant en outre un organe de verrouillage (600) coopérant avec l'organe de positionnement et de verrouillage pour verrouiller la manivelle mécanique en position de repos lorsqu'une tête de sonde est retirée du corps de sonde.

4. Ensemble de montage selon la revendication 2 ou la revendication 3, comprenant en outre un organe à ressort (605) exerçant une force sur l'organe de positionnement et de verrouillage pour tirer l'organe de positionnement et de verrouillage vers la position de verrouillage.

5. Ensemble de montage selon l'une quelconque des revendications 2 à 4, comprenant en outre un détecteur de position pour détecter une position angulaire de l'organe de positionnement et de verrouillage, la position angulaire correspondant à la position de verrouillage.

6. Ensemble de montage selon l'une quelconque des revendications 1 à 5, dans lequel l'organe de guidage de came et/ou l'organe de détrompage sont fixes par rapport au corps de sonde.

7. Ensemble de montage selon l'une quelconque des revendications 1 à 6, comprenant en outre des tiges flexibles de connexion électrique configurées pour établir des contacts électriques avec des patins de contact électrique d'une tête de sonde lorsque la tête de sonde est montée dans le corps de sonde.

8. Ensemble de montage pour une tête de sonde d'un dispositif portatif à ultrasons, l'ensemble de montage étant configuré pour coopérer avec l'ensemble de montage du corps de sonde selon l'une quelconque des revendications 1 à 6 et comprenant :
un organe de détrompage (225) identifiant une position de fixation de la tête de sonde ; et
une came (200) présentant une rainure de came (210) configurée pour coopérer avec un maneton d'un corps de sonde, la came étant mobile en rotation par rapport à l'organe de détrompage et étant solidarisée à un transducteur.

9. Dispositif portatif à ultrasons comprenant un corps de sonde avec un ensemble de montage selon l'une quelconque des revendications 1 à 7 et une tête de sonde avec un ensemble de montage selon la revendication 8, la tête de sonde étant détachable du corps de sonde.

10. Ensemble comprenant le dispositif portatif à ultrasons selon la revendication 9, la tête de sonde étant une première tête de sonde, l'ensemble comprenant en outre au moins une seconde tête de sonde, l'au moins une seconde tête de sonde comprenant un ensemble de montage selon la revendication 8, étant différente de la première tête de sonde, et pouvant être fixée au corps de sonde.

11. Ensemble selon la revendication 10, dans lequel l'au moins une seconde tête de sonde présente plusieurs transducteurs et/ou un type de transducteur différent de celui de la première tête de sonde.

12. Procédé de montage d'une tête de sonde dans un corps de sonde d'un dispositif portatif à ultrasons, le corps de sonde comprenant un ensemble de montage de corps de sonde comprenant un organe de guidage de came (300), un organe de détrompage de corps de sonde (305) et une manivelle mécanique rotative (315) comprenant un maneton (320), la manivelle mécanique rotative présentant une position de repos selon laquelle le maneton est aligné sur l'organe de guidage de came par rapport à une direction de montage d'une tête de sonde, la tête de sonde comprenant un ensemble de montage de tête de sonde comprenant un organe de détrompage de tête de sonde (225) identifiant une position de fixation de la tête de sonde et une came (200) présentant une rainure de came (210) configurée pour coopérer avec le maneton, la came étant mobile en rotation par rapport à l'organe de détrompage et étant solidarisée à un transducteur, le procédé comprenant
l'alignement (405) de la came et de l'organe de guidage de came ;
la mise en prise (410) de la came et de l'organe de guidage de came ;
l'alignement (415) des organes de détrompage ; et
la mise en prise (420) des organes de détrompage.

13. Procédé selon la revendication 12, dans lequel la mise en prise de la came et de l'organe de guidage de came comprend la mise en prise du maneton dans la rainure de came.

14. Procédé selon la revendication 12 ou la revendication 13, dans lequel la mise en prise des organes de détrompage comprend le déverrouillage de la manivelle mécanique.

15. Procédé de retrait d'une tête de sonde d'un corps de sonde d'un dispositif portatif à ultrasons, le corps de sonde comprenant un ensemble de montage de corps de sonde comprenant un organe de guidage de came (300), un organe de détrompage de corps de sonde (305) et une manivelle mécanique rotative (315) comprenant un maneton (320), la manivelle mécanique rotative présentant une position de repos selon laquelle le maneton est aligné sur l'organe de guidage de came par rapport à une direction de montage d'une tête de sonde, la tête de sonde comprenant un ensemble de montage de tête de sonde comprenant un organe de détrompage de tête de sonde (225) identifiant une position de fixation de la tête de sonde et une came (200) présentant une rainure de came (210) configurée pour coopérer avec le maneton, la came étant mobile en rotation par rapport à l'organe de détrompage et étant solidarisée à un transducteur, le procédé comprenant :
l'alignement (905) de la came et de l'organe de guidage de came ; et
la désolidarisation (915) de la tête de sonde du corps de sonde.

16. Procédé selon la revendication 15, dans lequel l'alignement de la came et de l'organe de guidage de came comprend la rotation de la manivelle mécanique et la détection d'une position angulaire prédéterminée de l'organe de positionnement et de verrouillage.

17. Procédé selon la revendication 15 ou la revendication 16, dans lequel la désolidarisation de la tête de sonde du corps de sonde comprend le verrouillage de la manivelle mécanique dans la position de repos selon laquelle le maneton est aligné sur l'organe de guidage de came.

18. Procédé de remplacement d'une première tête de sonde d'un dispositif portatif à ultrasons par une seconde tête de sonde, le dispositif portatif à ultrasons comprenant une tête de sonde et un corps de sonde, le procédé comprenant :
le retrait de la première tête de sonde du corps de sonde selon l'une quelconque des revendications 15 à 17 ; et
le montage de la seconde tête de sonde sur le corps de sonde selon l'une quelconque des revendications 12 à 14,
la seconde tête de sonde étant identique à la première tête de sonde ou présentant des caractéristiques de mesure par ultrasons différentes.
